(19) **Europäisches Patentamt**

**European Patent Office**

**Office européen des brevets**

(11) **EP 1 464 406 A2**

(12) **EUROPÄISCHE PATENTANMELDUNG**

(43) Veröffentlichungstag:
06.10.2004 Patentblatt 2004/41

(51) Int Cl.⁷: **B05B 7/00**, A45D 34/00

(21) Anmeldenummer: 04003256.7

(22) Anmeldetag: **13.02.2004**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **13.02.2003 DE 10306185**

(71) Anmelder:
• **Maurer, Michael, Dr.**
**84180 Loiching (DE)**
• **Christopulus, Antonios**
**84130 Dingolfing (DE)**

(72) Erfinder:
• **Maurer, Michael, Dr.**
**84180 Loiching (DE)**
• **Christopulus, Antonios**
**84130 Dingolfing (DE)**

(74) Vertreter: **Pausch, Thomas, Dipl.-Phys.**
**Patentanwalt**
**Olchinger Strasse 56**
**82194 Gröbenzell (DE)**

(54) **Vorrichtung und Verfahren zum dosierten Auftragen einer Zubereitung auf die Körperhaut eines Anwenders**

(57) Die Erfindung betrifft eine Vorrichtung und ein Verfahren zum dosierten Auftragen einer fluiden oder sprühfähigen Zubereitung auf die Körperoberfläche eines Anwenders, mit mehreren an unterschiedlichen Positionen angeordneten Verteilungselementen (ZD) für die gerichtete oder zumindest gebündelte Ausgabe der Zubereitung, und mit einer den Verteilungselementen (ZD) zugeordneten Steuerungseinrichtung (SPS), die die Verteilungselemente (ZD) im Sinne einer voreinstellbaren dosierten Abgabe der Zubereitung steuert. Für eine ultrafeine Zerstäubung und Vernebelung der Zubereitung bei minimalem Betriebsmittelverbrauch der Zubereitung ist ein Zerstäubungsmedium für den Transport der Zubereitung und für das Aufreissen des Flüssigkeitsstroms der Zubereitung in feinste Tropfen vorgesehen, wobei die mehreren Verteilungselemente (ZD) derart angeordnet sind, dass die Ausgabe der Zubereitung auf die Körperoberfläche des Anwenders mit einem vorbestimmten Vollkegelsprühbild mit optimalen Strahlwinkel (W) und Sprühweite (A) erfolgt.

Fig. 1

EP 1 464 406 A2

Printed by Jouve, 75001 PARIS (FR)

**Beschreibung**

Die Erfindung bezieht sich auf eine Vorrichtung sowie ein Verfahren zum dosierten Auftragen einer Zubereitung, insbesondere eines Körperpflegemittels wie Sonnenschutzmittel, auf die gesamte Körperoberfläche eines Anwenders.

Eine derartige Vorrichtung ist aus der DE G 87 14 652 bekannt geworden. Diese hat einen (einzigen) Vorratsbehälter und eine an einer Standsäule angebrachte Verteileinrichtung und mindestens eine horizontal angeordnete absperrbare Öffnung. Die gesamte Vorrichtung ist in einer durch eine Tür abschliessbare Kabine untergebracht. Für die Anwendung sind vom Anwender Absperrorgane zu betätigen, und die Sprühhöhe über Verstellelemente einzustellen. Das Aufsprühen wird durch Pumpen bewirkt.

Vom Anmelder angestellte Versuche haben ergeben, dass die in der DE G 87 14 652 beschriebene Vorrichtung nicht funktionsfähig ist. Ein Aufsprühen von Sonnenschutzmittel allein durch Pumpen ist nicht brauchbar im Sinne eines gewünschten gleichmäßigen Auftrages auf die Hautoberfläche und würde zu einem erheblichen und unwirtschaftlichen Sonnenschutzmittelverbrauch (grob geschätzt im 0,3 bis 0,5 Literbereich pro Anwendung) führen.

Der Erfindung liegt daher die Aufgabe zugrunde, eine wirtschaftlich und tehnisch brauchbare Vorrichtung und ein Verfahren anzugeben, mit welchem die automatisierte Auftragung einer fluiden oder sprühfähigen Zubereitung auf die gesamte Körperoberfläche eines Anwenders dergestalt gelingt, dass der Auftrag auf die Haut, Gesicht und Haare möglichst gleichmäßig verteilt, schonend und verlustarm gewährleistet werden kann.

Diese Aufgabe wird durch eine Vorrichtung gemäß Anspruch 1 und ein Verfahren gemäß Anspruch 10 gelöst.

Erfindungsgemäß ist eine Vorrichtung zum dosierten Auftragen einer fluiden oder sprühfähigen Zubereitung auf die gesamte Körperoberfläche eines Anwenders vorgesehen, mit mehreren an unterschiedlichen Positionen angeordneten Verteilungselementen für die gerichtete oder zumindest gebündelte Ausgabe der Zubereitung, und mit einer den Verteilungselementen zugeordneten Steuerungseinrichtung, die die Verteilungselemente im Sinne einer voreinstellbaren dosierten Abgabe der Zubereitung steuert, vorgesehen, wobei sich die Erfindung dadurch auszeichnet, dass für eine ultrafeine Zerstäubung und Vernebelung der Zubereitung bei minimalem Betriebsmittelverbrauch der Zubereitung ein Zerstäubungsmedium für das Aufreissen des Flüssigkeitsstroms der Zubereitung in feinste Tropfen vorgesehen ist, und die mehreren Verteilungselemente derart angeordnet sind, dass die Ausgabe der Zubereitung auf die Körperoberfläche des Anwenders mit einem vorbestimmten Vollkegelsprühbild mit optimalen Strahlwinkel und Sprühweite erfolgt. Eine dosierte Abgabe der Zubereitung bedeutet hierbei insbesondere auch, dass die Zubereitung aus mehreren Einzelkomponenten in den gewünschten Verhältnissen zusammengemischt und aufbereitet wird, wobei das Mischungsverhältnis der Einzelkomponenten oder die Dosierung im Falle einer lediglich aus einer Komponente bestehenden Zubereitung insbesondere vom Hauttyp des Anwenders, Lichtschutzfaktor, und/oder äußeren Faktoren wie aktuelle Sonnenstärke und dergleichen eingestellt wird.

Mit der Erfindung gelingt eine gleichmäßige, lückenlose, sparsame, angenehme Befeuchtung der Hautoberfläche, sowie von Gesicht und Haaren. Ein schnelles Trocknen der aufgebrachten Zubereitung nach der Anwendung ist gewährleistet, ausserdem sind keine zusätzlichen Tätigkeiten nach der Anwendung wie Abtrocknen, Verreiben usw notwendig. Die erfindungsgemäße Vorrichtung arbeitet vollautomatisch, es sind keinerlei manuellen Eingriffe notwendig wie bei der vorbekannten Vorrichtung. Mit der Erfindung gelingt der Schutzauftrag von Haut, Gesicht und Haaren mit nur einer Anwendung. Bei Verwendung eines textilneutralen Sonnenschutzöles ist die Anwendung mit Badebekleidung möglich. Eine abschließbare Kabine wie bei der vorbekannten Vorrichtung ist nicht notwendig. Ebensowenig sind bei der Erfindung keine aufwendigen Sensoren z. B. zur Körpergrößenermittlung oder zum Schutz von Gesicht oder Badebekleidung notwendig. Eine Körpergrößenerkennung ist nicht notwendig, da immer alle Düsen, unabhängig von der Körpergröße des Anwenders sprühen.

Dem Prinzip der Erfindung folgend ist die im weiteren auch als Flüssigkeit bezeichnete Zubereitung ein sprühfähiges Sonnenschutzmittel, insbesondere ein trockenes, nicht fettendes, wasserfestes Sonnenschuz-Öl, und das Zerstäubungsmedium ein unter Druck stehendes Gas, insbesondere Druckluft ist. Sonnenschutzcreme alleine ist nicht sprühfähig, nach der Efindung kommt daher ein Sonnenschutz-Öl oder allgemeiner ein sprühfähiges Sonnenschutzmittel bzw. Körperpflegemittel zum Einsatz.

Die erfindungsgemäße Vorrichtung kann als Standgerät ohne Kabine, aber ortsfest montiert oder auch transportabel, auch zerlegbar, oder auch in einer Sonderbauform, beispielsweise als integriertes Gerät, z.B. in Wände, Duschen, Umkleidekabinen, ausgebildet sein. Es benötigt lediglich eine 230 V AC Netz-Spannungsversorgung oder alternativ eine ortsunabhängige Fotovoltaik- oder Batterieversorgung.

Dem Prinzip der Erfindung folgend können die Verteilungselemente Zerstäuberdüsen sein, die vorzugsweise in einer einzigen Düsenreihe mit vertikal übereinander angeordneten Düsen vorgesehen sind. Hierbei sind die Verteilungselemente vorzugsweise 2-Stoff-Zerstäuberdüsen, wobei die Mischung der Zubereitung mit dem Zerstäubungsmedium unmittelbar innerhalb einer 2-Stoff-Zerstäuberdüse oder ausserhalb der Düse erfolgen kann. Die Düsen müssen nicht notwendigerweise alle gleichzeitig sprühen, sondern können dem Prinzip der Erfindung folgend untereinander zeitlich versetzt getaktet sprühen. Der Vorteil hierbei besteht in einer erheblichen Einsparung im Sonnenschutzmittelverbrauch.

Bei der besonders bevorzugten Ausführung der Erfindung erfolgt die Mischung der Zubereitung mit dem Zerstäubungsmedium nach dem Saugprinzip, bei dem die Zubereitung selbstansaugend aus einem Flüssigkeitsbehälter, dessen Niveau um eine gewisse Höhe tiefer als die Düsenöffnung liegt, dergestalt erfolgt, dass das Zerstäubungsmedium die Zubereitung aus dem tiefer liegenden Niveau ansaugt. Von Vorteil ist hierbei keine Pumpe(n) für die Flüssigkeitszuführung an die Düsen notwendig. Das Saugprinzip hat den Vorteil immer dann, wenn besonders geringe Mengen versprüht werden sollen; allerdings ist die Sprühmenge bei gleichem Zerstäubungsluftdruck stark von der Ansaughöhe anhängig. Als Lösung wird daher vorgeschlagen, für jede Düse einen separaten Vorratsbehälter zu verwenden. In Weiterführung der Erfindung ist somit für jede Düse ein separater Vorratsbehälter vorgesehen. Von Vorteil sind die Vorratsbehälter für die Zubereitung luftdicht abgeschlossene Kunststoffbeutel geringer Bauhöhe, die insbesondere liegend montiert sind. Ein flexibler Kunststoffbeutel als Behälter hat ausserdem den Vorteil, dass die Ansaughöhe bzw. das Ansaugniveau bei unterschiedlichen Beutelinhalt kaum variiert. Es ist keine Be- und Entlüftung notwendig, ausserdem gelingt ein schnelles Auswechseln der Beutel, z. B. über Schnellkupplungen.

Als Alternative kann die Mischung der Zubereitung mit dem Zerstäubungsmedium nach dem Nachströmprinzip erfolgen, bei dem die Zubereitung aus einem höher liegenden Flüssigkeitsniveau zugeführt und mit dem Zerstäubungsmedium gemischt wird. Der Nachteil dieser weniger bevorzugten Ausführung besteht aber darin, dass die Sprühmenge bei gleichem Zerstäubungsluftdruck stark vom hydrostatischem Vordruck (Vorratsbehälterinhalt) abhängig ist, und ausserdem Sonnenschutzöl bei undichten Ventilen auslaufen kann.

Als weitere Alternative kann die Mischung der Zubereitung mit dem Zerstäubungsmedium nach dem Druckprinzip erfolgen, bei dem die Zubereitung mit dem unter Druck stehenden Zerstäubungsmedium zugeführt und gemischt wird. Der Vorteil liegt hierbei darin, dass der Flüssigkeitsdruck und damit die Sprühmenge unabhängig vom Flüssigkeitsniveau für alle Düsen gleich ist. Die Verwendung eines zentralen Vorratsbehälters ist möglich. Als Nachteil dieser Alternative ist allerdings eine zusätzliche Pumpe notwendig.

Das erfindungsgemäße Verfahren zum dosierten Auftragen einer fluiden oder sprühfähigen Zubereitung auf die gesamte Körperoberfläche eines Anwenders sieht vor, dass für eine ultrafeine Zerstäubung und Vernebelung der Zubereitung bei minimalem Betriebsmittelverbrauch der Zubereitung ein Zerstäubungsmedium für die Zuführung der Zubereitung an die Verteilungselemente und zum Aufreissen des Flüssigkeitsstroms der Zubereitung in feinste Tropfen verwendet wird, und die mehreren Verteilungselemente derart angeordnet sind, dass die Ausgabe der Zubereitung auf die Körperoberfläche des Anwenders mit einem vorbestimmten Vollkegelsprühbild mit einem optimalen Strahlwinkel und Sprühweite erfolgt.

Das Zerstäubungsmedium dient der Zuführung und zum Aufreissen des Flüssigkeitsstroms in feinste Tropfen. Die Strömungsgeschwindigkeit und die Volumenstromdichte des Zerstäubungsmediums sind so gewählt, daß die aufgetragene Anwendung der Zubereitung als angenehm empfunden wird.

Die Zerstäubungsluft wird von einem kleinen Kompressor erzeugt, wobei der Kompressor optimal ausgelegt wird. Ein Druckluftspeicher und/oder Druckluftregelarmaturen sind nicht notwendig.

Alternativ kann die Zerstäubungsluft aus einer zentralen (z.B. hoteleigener) Druckluftversorgung, oder aus einer Druckluftflasche (z. B. nachfüllbare Sauerstoffflasche) stammen.

Vorteilhafte Weiterbildungen der Erfindung ergeben sich aus den weiteren Unteransprüchen.

Weitere Vorteile, Merkmale und Zweckmäßigkeiten der Erfindung ergeben sich aus der nachfolgenden Beschreibung eines Ausführungsbeispieles anhand der Zeichnung. Es zeigt:

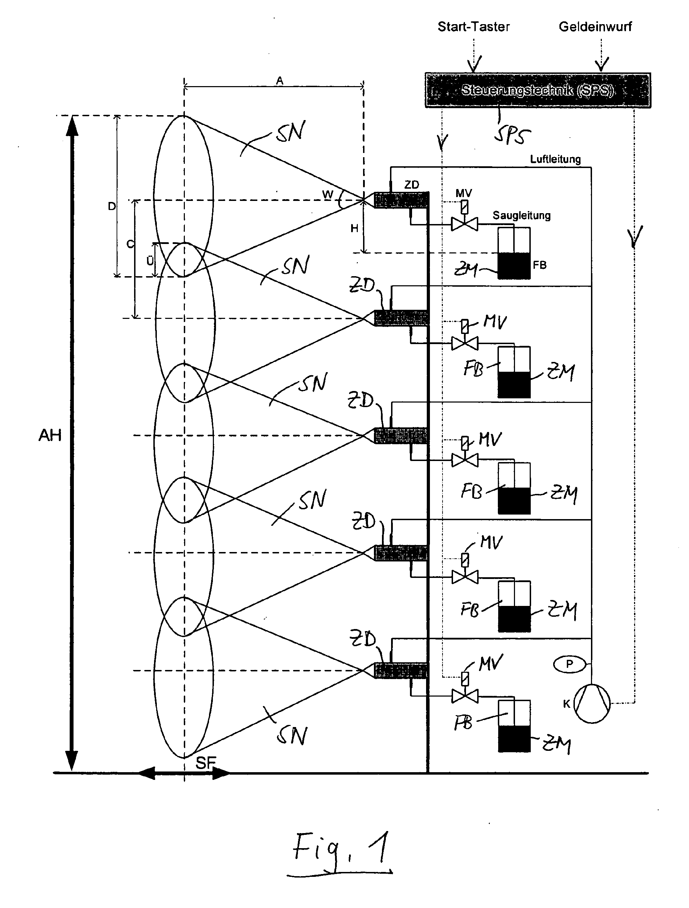

Fig. 1 eine schematische Ansicht eines Ausführungsbeispieles einer erfindungsgemäßen Vorrichtung zum dosierten Auftragen einer Zubereitung auf die Körperoberfläche eines Anwenders.

Das in Figur 1 dargestellte Ausführungsbeispiel einer erfindungsgemäßen Vorrichtung zum dosierten Auftragen einer fluiden oder sprühfähigen zubereitung auf die gesamte Körperoberfläche eines Anwenders umfasst mehrere an unterschiedlichen Positionen angeordnete Verteilungselemente (ZD) für die gerichtete oder zumindest gebündelte Ausgabe der Zubereitung, sowie eine den Verteilungselementen zugeordnete Steuerungseinrichtung mit Steuerungsblock (SPS), die die Verteilungselemente(ZD) im Sinne einer voreinstellbaren dosierten Abgabe der Zubereitung steuert. Für eine ultrafeine Zerstäubung und Vernebelung der Zubereitung bei minimalem Betriebsmittelverbrauch der Zubereitung ist ein Zerstäubungsmedium (ZM) für das Aufreissen des Flüssigkeitsstroms der Zubereitung in feinste Tropfen vorgesehen. Die mehreren Verteilungselemente (ZD) sind derart angeordnet, dass die Ausgabe der Zubereitung auf die Körperhaut des Anwenders mit einem vorbestimmten Vollkegelsprühbild mit optimalen, auch einstellbaren Strahlwinkel (W) und Sprühweite (A) erfolgt.

Das in Figur 1 dargestellte Ausführungsbeispiel kann unabhängig vom öffentlichen elektrischen Netzanschluss arbeiten, und hat zur elektrischen Leistungsversorgung eine Solaranlage mit Sonnenkollektoren bzw. Photovoltaikelementen, die die Vorrichtung mit elektrischer Leistung versorgen.

Zur Erzeugung eines feinen, homogenen Sprühnebels (SN) der Zubereitung werden spezielle 2-Stoff-Zerstäuberdüsen (ZD), geeignet für die Zerstäubung von Kleinstmengen, verwendet. Als Zerstäubungsmedium dient Druckluft. Die Druckluft wird von einem Kompressor (K) erzeugt. Die Zerstäubung (Mischung) von Flüssigkeit (Zubereitung) und Luft erfolgt im Inneren der Düsen (ZD) (Innenmischung). Die Zuführung der Flüssigkeit in die Düse erfolgt über das Saugprinzip (selbstansaugend) aus einem Flüssigkeitsbehälter (FB), dessen Niveau um die Höhe (H) tiefer als die Düsenöffnung liegt. Als Düsen-Strahlform wird ein Vollkegel mit einem Strahlwinkel (W) und einem sehr feinen und über eine weite Sprühdistanz homogenen Tropfenspektrum verwendet. Das erzeugte Strahlbild und die Vernebelungsmenge pro Zeiteinheit und pro Düse sind in erster Näherung eine Funktion der Düsenbauform, der Ansaughöhe (H), des Luftdrucks (Zerstäubungsdrucks) (P), des DruckluftVolumenstroms (V/t) und des Düsenöffnungswinkels (W).

Bei dem dargestellten Ausführungsbeispiel sind n=5 Stück einzelne Zerstäuberdüsen (ZD) vertikal in einem konstanten Abstand (C) übereinander angeordnet. Die gesamte Anwendungshöhe (AH) am Abstand (A) zu den Düsen ergibt sich aus der Anzahl (n) der Zerstäuberdüsen, des Düsenöffnungswinkels (W) und der Düsenüberlappung (Ü). Der Durchmesser des Vollkegels am Abstand (A) beträgt (D). Das Bezugszeichen (SF) kennzeichnet die Stellfläche des (nicht näher dargestellten) Anwenders während der Anwendung.

Ein exakt dimensionierter Miniaturkompressor (K) erzeugt im gesamten Pneumatiksystem im gewünschten Betriebspunkt einen konstanten Überdruck (P) mit einem Gesamtvolumenstrom (V/t), der sich gleichmäßig auf die n=5 Düsen verteilt. Mechanische Vibrationen und das Betriebsgeräusch des Kompressors sind minimal. Druck und Volumenstrom stehen sofort nach dem Starten des Kompressors zu Verfügung. Ein Druckbehälter als Speicher wird somit nicht benötigt. Auch sonstige Einstell- oder Regelarmaturen für das Druckluftsystem entfallen. Druckverluste im Druckluftverteilungssystem werden durch entsprechend dimensionierte Luftleitungsquerschnitte berücksichtigt. Der Luftdruck (P) kann an einem Präzisionsmanometer kontrolliert werden.

Als Flüssigkeitsbehälter (FB) werden luftdichte Kunststoffbeutel mit einer möglichst geringen Bauhöhe verwendet. Da die Flüssigkeitsbehälter liegend montiert sind, variiert die effektive Saughöhe (H) nur geringfügig. Eine damit verbundene Änderung der Sprühmenge kann vernachlässigt werden.

Jeder Düse oder Düsengruppe kann ein separates Magnetventil (MV) vorgeschaltet werden. Mit diesem Magnetventil wird die Saugleitung verschlossen oder geöffnet (notwendig für den getakteten Betrieb). Die Dimensionierung der Durchlassöffnungen der Magnetventile ist auf das Gesamtsystem abgestimmt. Bei laufendem Kompressor steht unmittelbar nach Öffnen des Magnetventils an der entsprechenden Düsenöffnung der Sprühnebel in der gewünschten Menge und Form zur Verfügung.

Die Logik der gesamten Ablaufsteuerung wird in einer Mikroprozessor-Steuerung (SPS) in Softwareform hinterlegt. Anzahl der benötigten Elektrokomponenten wird damit auf ein Minimum reduziert. Zudem besteht eine sehr hohe Flexibilität bezüglich Änderungen, Optimierungen oder Berücksichtigung von kundenspezifischen Sonderwünschen.

Nach dem Geldeinwurf oder anderweitiger Bezahlung (z. B. Kreditkarte) wird vom Anwender die Starttaste gedrückt. Gegebenenfalls kann über ein Display ein gewünschter Lichtschutzfaktor und andere Parameter ausgewählt werden. Mit einer Sekunde Verzögerung startet der Kompressor (K). Nach einer weiteren Sekunde wird das erste Magnetventil geöffnet und der Sprühvorgang beginnt. Die 5 Magnetventile werden in einer bestimmten, vorgegebnen Reihenfolge angesteuert und dabei getaktet. Anwendungsdauer, Ansteuerreihenfolge der Ventile und jeweiliger Arbeitstakt (Betriebs-/Pausezeiten) sind so gewählt, daß das erzeugte Sprühbild eine optimale, gleichmäßige Befeuchtung des Anwenders garantiert. Gleichzeitig wird ein minimaler Verbrauch des Sonnenschutzmittels erreicht. Nach dem Sprühvorgang läuft der Kompressor noch eine Sekunde nach und entleert dadurch das Düsensystem. Der Automat ist sofort für die nächste Anwendung bereit.

Die Anzahl der Sprühvorgänge kann am Display und/oder an der Steuerungseinrichtung SPS abgelesen werden. Dadurch können Sprühmittelverbrauch und Geldeinnahmen kontrolliert werden. Der Zähler ist gegen unberechtigten Reset geschützt.

Alle Verfahrensparameter sind aufeinander abgestimmt. Dadurch ist gewährleistet, daß bei minimalen Sprühmittelverbrauch ein optimales Sprühergebnis erzielt wird. Nach Rücksprache können die verfahrensparameter nach Kundenwunsch geändert werden.

In einer einfacheren Ausführungsform, z.B. für den Privatgebrauch wäre auch eine Ausführung denkbar, bei der die Steuerungseinrichtung nur einen Ein-Ausschalter aufweist.

Die erfindungsgemäße Vorrichtung verbraucht pro Anwendung ca. 15 ml Sonnencreme. Bei 5 Düsen entspricht dies 3 ml Sonnencremeverbrauch pro Düse und pro Anwendung. Die Vernebelung solcher Kleinstmengen gelingt vorzugsweise mit Zweistoffdüsen. Zugleich erfolgt die Flüssigkeitszuführung vermittels dem Saugprinzip (selbstansaugend, aus einem tiefer liegenden Flüssigkeitsniveau). Jede Düse ist mit einem eigenen, separaten Vorratsbeutel verbunden. Jede Düse versprüht pro Anwendung beispielsweise 3 ml Sonnenschutzmittel. Es können Vorratsbeutel mit verschiedenen Inhaltsvolumen eingesetzt werden. Beispielsweise:

1000 ml - Beutel = 333 Anwendungen

1500 ml - Beutel = 500 Anwendungen

2000 ml - Beutel = 666 Anwendungen.

Die angegebenen Zahlenwerte dienen der Erläuterung und können selbstverständlich variieren.

Bei dem in Figur 1 dargestellten Ausführungsbeispiel der Erfindung ist die gesamte Vorrichtung in offener Form, d.h. ohne geschlossene Kabine, betrieben; zusätzlich kann ein geeigneter Sichtschutz oder Windschutz vorgesehen sein.

Im dargestellten Ausführungsbeispiel ist nur eine Düsenreihe mit vertikal angeordneten Düsen vorgesehen. Alternativ wären zwei oder auch mehrere Düsenreihen möglich. Der Anwender braucht sich dann während der Anwendung nicht zu drehen. Der Nachteil liegt aber in einem höheren Sonnenschutzmittelverbrauch, was wirtschaftlich ungünstiger ist.

**Patentansprüche**

1.  Vorrichtung zum dosierten Auftragen einer fluiden oder sprühfähigen Zubereitung auf die Körperoberfläche eines Anwenders, mit mehreren an unterschiedlichen Positionen angeordneten Verteilungselementen (ZD) für die gerichtete oder zumindest gebündelte Ausgabe der Zubereitung, und mit einer den Verteilungselementen (ZD) zugeordneten Steuerungseinrichtung (SPS), die die Verteilungselemente (ZD) im Sinne einer voreinstellbaren dosierten Abgabe der Zubereitung steuert, **dadurch gekennzeichnet, dass** für eine ultrafeine Zerstäubung und Vernebelung der Zubereitung bei minimalem Betriebsmittelverbrauch der Zubereitung ein Zerstäubungsmedium für den Transport der Zubereitung und für das Aufreissen des Flüssigkeitsstroms der Zubereitung in feinste Tropfen vorgesehen ist, und die mehreren Verteilungselemente (ZD) derart angeordnet sind, dass die Ausgabe der Zubereitung auf die Körperoberfläche des Anwenders mit einem vorbestimmten Vollkegelsprühbild mit optimalen Strahlwinkel (W) und Sprühweite (A) erfolgt.

2.  Vorrichtung nach Anspruch 1, **dadurch gekennzeichnet, dass** die Zubereitung ein sprühfähiges Sonnenschutzmittel, insbesondere ein trockenes, nicht fettendes, wasserfestes Sonnenschutz-Öl ist, und das Zerstäubungsmedium ein unter Druck stehendes Gas, insbesondere Druckluft ist.

3.  Vorrichtung nach Anspruch 1 oder 2, **dadurch gekennzeichnet, dass** die Verteilungselemente (ZD) Zerstäuberdüsen sind, die in einer einzigen Düsenreihe mit vertikal übereinander angeordneten Düsen vorgesehen sind.

4.  Vorrichtung nach einem der vorhergehenden Ansprüche, **dadurch gekennzeichnet, dass** die Verteilungselemente 2-Stoff-Zerstäuberdüsen sind, wobei die Mischung der Zubereitung mit dem Zerstäubungsmedium unmittelbar innerhalb einer 2-Stoff-Zerstäuberdüse oder ausserhalb der Düse erfolgt.

5.  Vorrichtung nach einem der vorhergehenden Ansprüche, **dadurch gekennzeichnet, dass** die Düsen (ZD) untereinander zeitlich versetzt getaktet sprühen.

6.  Vorrichtung nach einem der vorhergehenden Ansprüche, **dadurch gekennzeichnet, dass** die Mischung der Zubereitung mit dem Zerstäubungsmedium nach dem Saugprinzip erfolgt, bei dem die Zubereitung selbstansaugend aus einem Flüssigkeitsbehälter (FB), dessen Niveau um eine gewisse Höhe (H) tiefer als die Düsenöffnung liegt, dergestalt erfolgt, dass das Zerstäubungsmedium die Zubereitung aus dem tiefer liegenden Niveau ansaugt, wobei für jede Düse ein separater Vorratsbehälter vorgesehen ist.

7.  Vorrichtung nach einem der vorhergehenden Ansprüche, **dadurch gekennzeichnet, dass** die Vorratsbehälter (FB) für die Zubereitung luftdicht abgeschlossene Kunststoffbeutel sind, die insbesondere liegend montiert sind.

8.  Vorrichtung nach einem der vorhergehenden Ansprüche, **dadurch gekennzeichnet, dass** jeder Düse oder Düsengruppe ein separates Magnetventil (MV) vorgeschaltet ist, mit welchem die der Düse zugeordnete Saugleitung selbsttätig verschlossen oder geöffnet wird.

9.  Vorrichtung nach einem der vorhergehenden Ansprüche, **dadurch gekennzeichnet, dass** die Mischung der Zubereitung mit dem Zerstäubungsmedium nach dem Nachströmprinzip erfolgt, bei dem die Zubereitung aus einem höher liegenden Flüssigkeitsniveau zugeführt und mit dem Zerstäubungsmedium gemischt wird, oder die Mischung der Zubereitung mit dem Zerstäubungsmedium nach dem Druckprinzip erfolgt, bei dem die Zubereitung mit dem unter Druck stehenden Zerstäubungsmedium zugeführt und gemischt wird.

10. Verfahren zum dosierten Auftragen einer fluiden oder sprühfähigen Zubereitung auf die Körperoberfläche eines Anwenders, mit mehreren an unterschiedlichen Positionen angeordneten Verteilungselementen (ZD) für die gerichtete oder zumindest gebündelte Ausgabe der Zubereitung, und mit einer den Verteilungselementen (ZD) zugeordneten Steuerungseinrichtung (SPS), die die Verteilungselemente (ZD) im Sinne einer voreinstellbaren dosierten Abgabe der Zubereitung steuert, **dadurch gekennzeichnet, dass**
für eine ultrafeine Zerstäubung und Vernebelung der Zubereitung bei minimalem Betriebsmittelverbrauch der Zubereitung ein Zerstäubungsmedium für den Transport der Zubereitung und
für das Aufreissen des Flüssigkeitsstroms der Zubereitung in feinste Tropfen verwendet wird, und die mehreren Verteilungselemente derart angeordnet sind, dass die Ausgabe der Zubereitung auf die Körperoberfläche des Anwenders mit einem vorbestimmten Vollkegelsprühbild mit optimalen Strahlwinkel und Sprühweite erfolgt.

# EP 1 464 406 A2

Fig. 1